Europäisches Patentamt

European Patent Office

Office européen des brevets

(11) Veröffentlichungsnummer: **0 315 982**
**A2**

## (12) EUROPÄISCHE PATENTANMELDUNG

(21) Anmeldenummer: **88118714.0**

(22) Anmeldetag: **10.11.88**

(51) Int. Cl.⁴: **A61M 25/00**

(30) Priorität: **12.11.87 DE 3738428**

(43) Veröffentlichungstag der Anmeldung:
**17.05.89 Patentblatt 89/20**

(84) Benannte Vertragsstaaten:
**AT BE CH DE ES FR GB GR IT LI LU NL SE**

(71) Anmelder: **Solzbach, Ulrich, Dr.**
**Spechtweg 15**
**D-7800 Freiburg(DE)**

Anmelder: **Wollschläger, Helmut, Dr.**
**Laufenerstrasse 15**
**D-7800 Freiburg(DE)**

(72) Erfinder: **Solzbach, Ulrich, Dr.**
**Spechtweg 15**
**D-7800 Freiburg(DE)**
Erfinder: **Wollschläger, Helmut, Dr.**
**Laufenerstrasse 15**
**D-7800 Freiburg(DE)**

(74) Vertreter: **Schmitt, Hans, Dipl.-Ing. et al**
**Patentanwälte Dipl.-Ing H. Schmitt Dipl.-Ing.**
**W. Maucher Dreikönigstrasse 13**
**D-7800 Freiburg(DE)**

(54) **Katheter.**

(57) Ein erfindungsgemäßer Katheter (1) dient zur Wärme-Nachbehandlung eines im Bereich einer Stenose (18) dilatierten Gefäßabschnittes.
Dazu weist der Katheter (1) einen dilatierbaren Ballon (2) auf, der in einem Teilbereich seiner Wandung mit einem Widerstand-Heizelement (6) ausgebildet ist. Dieses Heizelement ist durch eine elektrisch leitende Heizfolie (1o) gebildet, die mit gegenüberliegenden Längskanten an Stromversorgungs-Drähte (7) angeschlossen ist. Diese Drähte (7) sind innerhalb des Katheterschaftes (3) nach außen geführt und mit einer Stromquelle (8) verbunden. Durch die Wärme-Nachbehandlung nach einer Dilatation kann eine Restenosierung beseitigt und Dissektionen "verschweißt" werden.

Fig 1

## Katheter

Die Erfindung betrifft einen Katheter mit einem am Endbereich befindlichen, dilatierbaren Ballon, der elektrisch leitende Teilbereiche aufweist und elektrisch heizbar ist, zum Erwärmen von dilatierten Gefäßabschnitten.

In der Medizin, speziell in der Radiologie und Kardiologie, ist die Dilatation (Aufdehnung) von Stenosen (Verengungen) entlang von Arterien eine inzwischen oft benutzte und weitgehend erfolgreiche Methode, um den Blutfluß und damit die Sauerstoff- und Energieversorgung im periphären Gefäßbett zu erhöhen.

Dabei wird ein sogenannter Dilatationskatheter, an dessen vorderen Ende sich ein aufblasbarer Ballon befindet, in das entsprechende stenosierte Gefäßsegment vorgeschoben.

Eine Schwierigkeit bei dem bisherigen Dilatationsverfahren ist das Auftreten von durch die Dilatation verursachten Dissektionen (Risse) in der Gefäßwand, was als Hauptursache für anschließende, plötzliche Gefäßverschlüsse angesehen wird. Weiterhin kann eine Restenosierung durch die elastischen Eigenschaften des dilatierten Wandmateriales auftreten.

Durch intraarterielle Anwendung von Laser-Strahlen wurden bereits Versuche gemacht, die Stenosen durch lokale Anwendung von Wärme zu behandeln. Beispielsweise ist durch die Veröffentlichung von "Cumberland, D.C., Starkey, I.R. et al.: Percutanous Laser-Assisted Coronary Angioplasty. Lancet 2, 214 (1986)" bereits die Anwendung eines Lasers bei der Behandlung von Stenosen bekannt. Der an sich bewährte Einsatz eines Lasers hat jedoch den Nachteil, daß hierzu ein Aufwand erforderlich ist, der den Einsatz auf größere klinische Zentren beschränkt.

Es wurde deshalb auch schon diskutiert, das dilatierte Gefäß durch Einbringen von Wärme mittels induktiver Einkopplung von Wirbelströmen oder durch ein Hochfrequenz-Strömungsfeld zu koagulieren (vgl. z.B. Biomedizinische Technik Band 32, Ergänzungsband, Sept. 1987 "HF-Energie gestützte Ballondilatation" von R. Thull, M. Hubmann und Biomedizinische Technik Band 32, Ergänzungsband, Sept. 1987 "Untersuchung der Hochfrequenzkoagulation von Gefäßpräparaten durch flächig applizierte Elektroden" von M. Hubmann, A. Weikl, H.-J.Pesch und R. Thull).

Die induktive Erwärmung soll dabei durch Einkopplung des elektrischen Feldes einer Primärspule in einen als Kurzschlußwicklung wirkenden Metallfilm auf den Ballon eines Dilatationskatheters erfolgen. Berechnungen haben jedoch ergeben, daß bei einer induktiven Erwärmung praktisch nicht realisierbare Hochfrequenzleistungen erforderlich wären.

In Versuchen an Gefäßpräparaten wurde auch bereits eine Erwärmung durch Hochfrequenzapplikation durchgeführt, die aber bislang über das Experimentierstadium nicht hinaus gingen. Die speziellen Probleme bei der klinischen Anwendung am Patienten, z. B. die Hochfrequenzenergieübertragung zu unterschiedlichen Stellen im menschlichen Körper und somit unterschiedliche Eindringtiefen, die genaue Einstellung bezüglich Intensität und Positionierung und dgl. konnten somit noch gar nicht erfaßt werden.

Aus der EP 0 182 689 ist es ebenfalls bereits bekannt, als Heizquelle ein Laser vorzusehen. Weiterhin wird in einer Ausführungsvariante vorgeschlagen, daß auf der Oberfläche der Ballonhülle Widerstandsdraht in Form eines Maschennetzes aufgebracht ist. Nachteilig ist hierbei, daß mit einem solchen Draht-Maschennetz keine homogene Wärmeapplikation in dem erwünschten Maße möglich ist, da hierbei zwischen den einzelnen Drähten Zonen geringerer Erwärmung vorhanden sind. Dies ist medizinisch nicht erwünscht, da an den Stellen des heißen Drahtes Koagulationsnekrosen hervorgerufen werden können, während benachbart die Maschenbereiche nicht direkt erwärmt werden, so daß dort die zum "Verschweißen" der Arterienwand notwendige Temperatur nicht erreicht wird. Ein weiterer Nachteil des Drahtgeflechtes besteht auch in seiner Bruchempfindlichkeit. Dabei muß berücksichtigt werden, daß die vewendeten Drähtchen einerseits sehr dünn sein müssen, um die Aufdehn- und Zusammenfaltbewegungen des Ballons mitmachen zu können und um diese Bewegungen möglichst wenig zu behindern, andererseits führt dies aber zu einem sehr bruchempfindlichen Heizgeflecht.

Aufgabe der vorliegenden Erfindung ist es, einen Ballonkatheter zu schaffen, mit dem eine thermische Nachbehandlung eines dilatierten Gefäßabschnittes mit vergleichsweise geringem Aufwand durchführbar ist und mit dem die Wärmeapplikation genau positionierbar und dosierbar und bezüglich der Intensität auch gut steuerbar ist. Auch soll eine möglichst homogene Wärmeapplikation erfolgen. Schließlich soll eine einfach Handhabung möglich sein.

Zur Lösung dieser Aufgabe wird erfindungsgemäß insbesondere vorgeschlagen, daß das Heizelement eine elektrisch leitende Heizfolie aus Kunststoff ist, die nach außen, der Innenseite eines Gefäßabschnittes zugewandt, sowie nach innen elektrisch isoliert ist.

Durch den Einsatz eines solchen Heizelementes wird eine praktisch homogene Wärmeapplikation erreicht. Außerdem ist hierbei vorteilhaft, daß die flexible Heizfolie die Bewegungen des Ballons (aufweiten, zusammenknittern) ohne weiteres mitmachen kann, ohne daß dabei eine Bruchgefahr besteht. Trotz dieser Bewegbarkeit bleiben die Heizeigenschaften voll erhalten. Die äußere Isolierung vermeidet einen Strombypass zur Heizfolie und außerdem wird dadurch auch die Gefahr von Komplikationen, wie z. B. Herz-Rhythmusprobleme vermieden. Die innere Isolierung verhindert, daß beim Zusammenfalten des Ballons leitende Teile der Heizfolie innen aneinander gelangen, wodurch Bereiche höherer Leitfähigkeit vorhanden wären.

Außerdem ist ein solcher Katheter mit vergleichsweise geringem Aufwand, gleichzeitig jedoch unter Berücksichtigung der im medizinischen Bereich vorgesehenen Sicherheitsanforderungen herstellbar. Die bei im Gebrauch befindlichen Dilatations-Ballonkathetern vorhandenen und bewährten Grund-Konstruktionsdetails können auch bei dem erfindungsgemäßen Katheter in vorteilhafter Weise beibehalten werden.

Eine bevorzugte Ausführungsform sieht vor, daß die Heizzone des Ballons etwa in einem mittleren Bereich seiner Längserstreckung und mit Abstand zu seinen Enden vorgesehen ist und sich vorzugsweise über etwa zei Drittel der Gesamtlänge erstreckt.

Somit werden die Enden des Ballons nicht oder weniger beheizt, so daß dort anstehendes Blut nicht erwärmt wird und somit die Gefahr einer dadurch bedingten Thrombenbildung auch nicht vorhanden ist.

Zweckmäßigerweise sind als galvanische Verbindung zwischen der äußeren Stromquelle und dem Heizelement Stromversorgungs-Drähte vorgesehen, die vorzugsweise eine äußere Isolierung aufweisen und vorzugsweise innerhalb des Katheterschaftes geführt sind und daß insbesondere zwei oder gegebenenfalls auch eine davon vielfache Anzahl von Drähten oder dgl. in der Katheterschaftwand integriert untergebracht sind. Über diese Drähte läßt sich eine elektrisch gut leitende Verbindung zwischen dem Heizelement und der äußeren Stromquelle herstellen, wobei diese Drähte auch hinreichend dünn und elastisch ausgebildet sein können, andererseits in Abstimmung an die vorhandenen, beengten Platzverhältnisse und auch an die erforderliche Verformbarkeit während der Katheterpositionierung.

Durch die äußere Isolierung kann gefahrlos auch an kritischen Stellen mit dem Katheter gearbeitet werden, insbesondere im Koronarbereich. Durch die Unterbringung der Drähte im Katheterschaft bleibt die gute Handhabbarkeit des Katheters erhalten. Wenn mehrere Drähte in der Katheterschaftwand integriert untergebracht sind, treten diese innerhalb des verlegten Katheters nicht störend in Erscheinung und reduzieren auch nicht das Katheterlumen.

Eine andere Ausführungsform sieht vor, daß als galvanische Verbindung Folienbänder aus elektrisch leitendem Material, z. B. aus Metallfolie oder aus leitender Kunststoffolie an oder in der Katheterschaftwand vorgesehen sind.

Bei Verwendung von elektrisch leitenden Kunststoffolien können diese auch direkt in den Bereich des Heizelementes übergehen, wobei durch Variation des spezifischen Widerstandes in dem oder den vorgesehenen Bereichen Heizzonen geschaffen werden können.

Nach einem weiteren Vorschlag der Erfindung ist vorgesehen, daß der Katheter als Heizballon-Katheter und gleichzeitig als Aufweitballon-Katheter ausgebildet und insbesondere bezüglich des Aufweitdruckes auf die Anforderungen beim Aufweiten bemessen ist.

Bei dieser Ausführungsform kann mit einem einzigen Ballon-Katheter zunächst dilatiert und anschließend kann, ohne Auswechseln des Katheters, der liegende Katheter auch zum Wärmebehandeln der Dilatationsstelle verwendet werden. Während der Dilatation kann wie üblich mit den vorgesehenen Aufweitdrücken gearbeitet werden, während bei der Nachbehandlung mit Wärme wesentlich geringere Drücke vorgesehen sind, die so bemessen sind, daß die Außenseite des Ballons einen innigen Kontakt mit dem Endothel hat. Anstatt die Dilatation und die Wärmebehandlung nacheinander vorzunehmen, besteht auch die Möglichkeit, daß gleichzeitig mit dem Aufweiten auch schon die Wärmebehandlung erfolgt.

Zusätzliche Ausgestaltungen der Erfindung sind in den weiteren Unteransprüchen aufgeführt. Nachstehend ist die Erfindung mit ihren wesentlichen Einzelheiten anhand der Zeichnungen in Ausführungsbeispielen näher erläutert.

Es zeigt zum Teil stärker schematisiert:

Fig. 1 einen heizbaren Ballon-Katheter mit angeschlossenen Bedienungs- und Überwachungsapparaturen,

Fig. 2 eine schematische Darstellung eines dilatierten Ballons mit Heizelement,

Fig. 3 und 4 Teilschnittansichten eines Ballons mit unterschiedlicher Anordnung einer Heizschicht,

Fig. 5 einen Querschnitt eines Katheterschaftes mit darin integrierten Zuleitungsdrähten,

Fig. 6 einen Querschnitt eines Katheterschaftes mit in dessen Wandung integrierten Metallfolien oder dgl.,

Fig. 7 und 8 schematisierte Schnittdarstellungen gemäß den Schnittlinien IX-IX und X-X in Fig. 1.

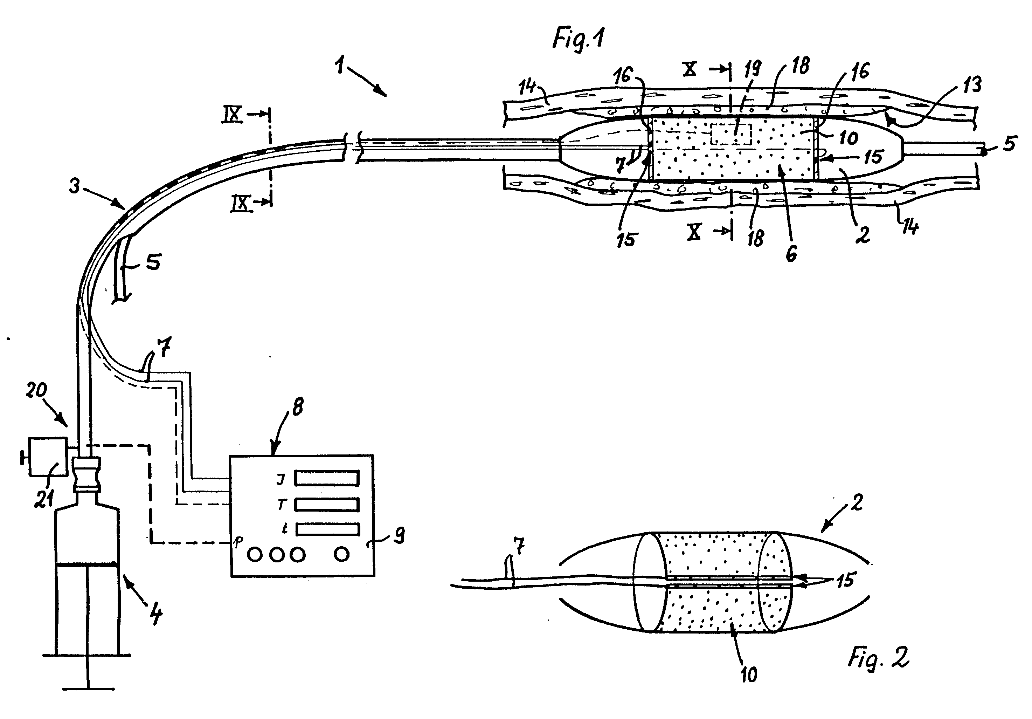

Ein in Fig. 1 gezeigter Katheter 1 weist an seinem einen Ende (distal) einen dilatierbaren Ballon 2 auf, an den sich ein Katheterschaft 3 anschließt. Am äußeren Ende des Katheterschaftes 3 ist eine Spritze 4 angeschlossen, mittels der dem Ballon 2 ein Dilatationsmedium zum Aufweiten zugeführt werden kann. Außerdem erkennt man in Fig. 1 noch einen Führungsdraht 5, der als erstes bis weit distal einer zu behandelnden Stenose 18 in das Blutgefäß eingeführt wird. Der Katheter 1 wird anschließend über diesen Draht 5 eingeführt und mit seinem Ballon 2 bis zu der Stenose vorgeschoben.

Dies entspricht der üblichen Kaltenbach-Technik. Gegebenenfalls kann auch mit einer anderen Technik, beispielsweise nach dem Monrail-Bonzel-Prinzip gearbeitet werden.

Der Ballon 2 weist ein Widerstands-Heizelement auf, das über Drähte 7 oder dgl. mit einer äußeren Stromquelle 8 verbunden ist. Die Stromquelle kann dabei innerhalb eines Steuer- oder Überwachungsgerätes 9 untergebracht sein. Das Heizelement 6 ist vorzugsweise als Flachkörper ausgebildet und im Wandungsbereich des Ballongs 2 angeordnet. Das Heizelement ist durch eine elektrisch leitende Heizfolie 10 gebildet, deren Leitfähigkeit beispielsweise durch Zusatz eines elektrisch leitenden Materials, wie Kohlenstoff oder dgl. erreicht wird.

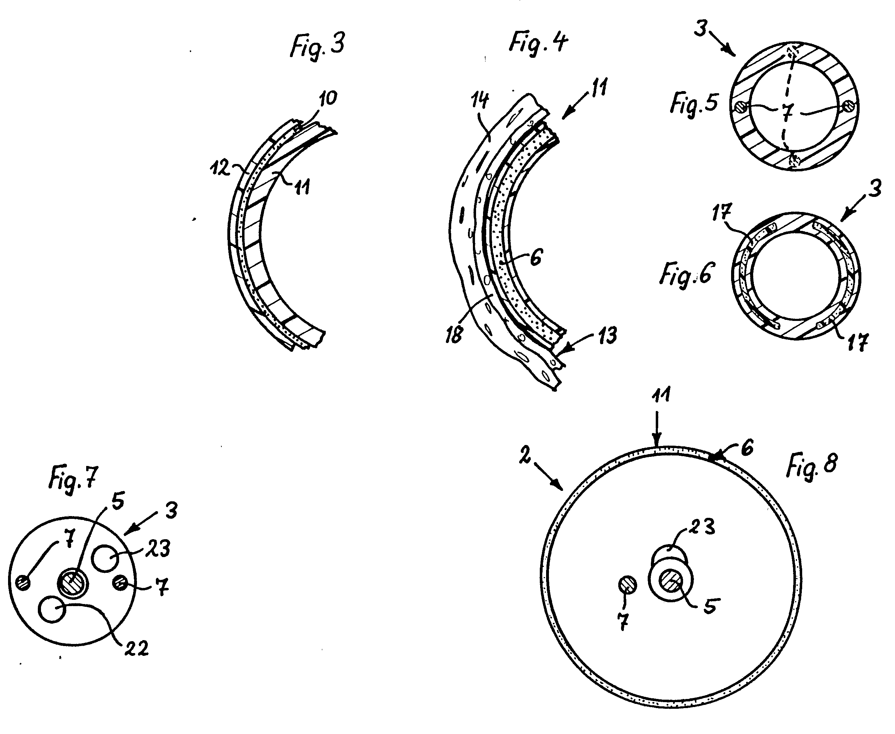

Die "heizaktiven" Bereiche sind in den Figuren punktiert gekennzeichnet. In den Fig. 3 u. 4 ist erkennbar, daß die Heizfolie 10 unterschiedlich im Wandungsbereich des Ballons 2 angeordnet sein kann. Gemäß Fig. 3 ist die Heizfolie 10 außenseitig auf der Wandung 11 angebracht und außenseitig durch eine Schicht 12 elektrisch isoliert.

Für eine möglichst direkte Wärmeübertragung zwischen der Heizfolie 10 und der Innenseite 13 eines Gefäßes 14 im Bereich einer Stenose (vgl. Fig. 4) ist die Anbringung der Heizfolie 10 außenseitig auf der Wandung 11 des Ballons 2 vorteilhaft. Bevorzugt ist dabei vorgesehen, daß die Heizfolie selbst die Ballonwand bildet, wobei sie zumindest außenseitig eine Isolierschicht hat. In Fig. 4 ist eine dreischichtige Ausbildung einer gleichzeitig die Wandung 11 bildenden Heizfolie gezeigt.

Der "heizaktive" Bereich erstreckt sich nur über einen Teilbereich des Ballons, zweckmäßigerweise über dessen Mittelbereich, wie dies in den Figuren 1 bis 3 erkennbar ist. Beispielsweise kann sich dieser Heizbereich über zwei Drittel der Gesamtlänge des Ballons 2 erstrecken, wobei die Endbereiche des Ballons jeweils nicht beheizt sind. Dadurch wird ein direkter Kontakt des Heizbereiches mit dem vorder- und rückseitig des Ballons 2 anstehenden Blut vermieden. Dementsprechend würde bei einer Ausführung der Ballon-Wandung 11 gemäß Fig. 4 die innere Heizfolienschicht mit Abstand zu den Ballonenden aufhören, wobei dann die beiden Außenschichten zusammengeführt sein können.

Die Stromversorgungs-Drähte 7 sind zweckmäßigerweise an gegenüberliegenden Endbereichen des Widerstand-Heizelementes 6, insbesondere der Heizfolie 10 angeschlossen, wie dies gut in den Figuren 1 u. 2 erkennbar ist. Für eine gleichmäßige Stromverteilung bei den Anschlußstellen 15 (Fig. 1) sind die Anschlußendbereiche der Heizfolie 10 mit größerer spezifischer Leitfähigkeit als die Heizfolie selbst ausgebildet. In Fig. 1 ist die Heizfolie 10 als geschlossener Ringmantel mit in Umfangsrichtung des Ballons 2 umlaufenden Rändern 16 ausgebildet, die als Anschlüsse für die Stromversorgungsdrähte 7 dienen und durch ihre hohe spezifische, elektrische Leitfähigkeit für eine entsprechend gleichmäßige Stromzuführung über den Umfang der Heizfolie 10 sorgen.

Bei der Ausführungsform gemäß Fig. 2 ist die Heizfolie 10 als offener Ringmantel mit in Längsrichtung des Ballons 2 verlaufenden, voneinander getrennten Längskanten, die ihrerseits wiederum die Anschlüsse für die Stromversorgungs-Drähte 7 bilden, ausgebildet. Auch hierbei weisen diese Längskanten eine spezifisch hohe Leitfähigkeit auf. Beispielsweise kann dies auch erreicht werden, indem die Drähte 7 entlang dieser Längskanten weitergeführt sind. Schließlich besteht auch noch die Möglichkeit, daß von den Enden der Stromversorgungs-Drähte 7 ausgehend, eine Vielzahl von Drähten zu ringförmigen Anschlußstellen an den Enden der Heizfolie 10 führen. Die Enden der Drähtchen sind dabei vorzugsweise in gleichmäßigen Abständen an die Ring-Anschlußstellen 15 der Heizfolie lO angeschlossen. Anstatt dieser Drähtchen könnte auch Leitfolie vorgesehen sein.

Die Stromversorgungs-Drähte 7 sind innerhalb des Katheterschaftes 3 geführt, wobei sie zweckmäßigerweise, wie in Fig. 5 erkennbar, in der Katheterschaftwand integriert untergebracht sind. Um den Widerstand der Zuleitung zu dem Heizelement 6 möglichst gering zu halten und auch unter Berücksichtigung der beengten Platzverhältnisse bei dem Katheterschaft 3, können auch mehr als zwei Drähte 7 vorgesehen sein, wobei dann jeweils die Hälfte der Anzahl parallel geschaltet sind. In Fig. 5 sind zwei zusätzliche Drähte strichliniert angedeutet.

Andererseits besteht aber auch die Möglichkeit, daß anstatt von Drähten 7 Folienbänder 17 aus elektrisch leitendem Material, z. B. aus Metallfolie oder aber Kunststoff-Leitfolie als galvanische Verbindung zwischen der äußeren Stromquelle 8 und dem Heizelement 6 vorgesehen sind. Auch diese Folienbänder sind zweckmäßigerweise in die Katheterschaftwand integriert, wie dies in Fig. 6 angedeutet ist.

Mit Hilfe des erfindungsgemäßen Katheters 1 kann nach einer Dilatation im Bereich einer Stenose 18 (vgl. Fig. 1) eine Wärmebehandlung in diesem Bereich vorgenommen werden, durch die insbesondere mittels Denaturierung der Eiweißstrukturen ein "Verschweißen" von möglichen Disskētionen bzw. ein "Erstarren" der Gefäßwand erreicht werden kann.

Bei Behandlung einer Stenose 18 kann zunächst in üblicher Weise durch Einbringen eines Dilatations-Katheters die Stenose aufgeweitet werden. Anschließend kann nach dem Zurückziehen des Dilatationskatheters der erfindungsgemäße Katheter 1 über den noch liegenden Führungsdraht 5 nachgeschoben und mit seinem Ballon 2 im zuvor dilatierten plaziert werden. Da nun nicht eine nochmalige Dilatation vorgesehen ist, genügt ein wesentlich geringerer Aufblasdruck als bei dem zuvor eingeführten Dilatationskatheter. Beispielsweise könnte hierbei ein Zehntel des Dialtationsdruckes genügen.

Es soll damit nur sichergestellt sein, daß ein inniger Kontakt zwischen Ballon-Außenseite und dem Endothel des Gefäßes erreicht wird.

Bei dieser Wärme-Nachbehandlung sollen insbesondere die durch die Dilataion entstandenen Schäden (Risse, Quetschphänomente) behandelt, insbesondere koaguliert werden.

Die Arbeitstemperatur soll dabei etwa 60° bis 90°, vorzugsweise etwa 70° betragen. Die gewählte Temperatur hängt dabei auch von der vorgesehenen Einwirkdauer ab.

Um das zweimalige Einführen eines Katheters mit den damit verbundenen Umtänden und dem Zeitaufwand zu vermeiden, kann der erfindungsgemäße Katheter 1 als Heizballon-Katheter und gleichzeitig als Aufweitballon-Katheter ausgebildet sein. Bezüglich seiner Festigkeit ist dabei dieser Kombinations-Katheter auf die höheren Druckbelstungen beim Aufweiten bemessen. Durch diese Ausbildung kann in einem Arbeitsgang dilatiert werden und unmittelbar danach die Wärmebehandlung erfolgen.

Für eine möglichst geringe Wärmeableitung von dem Heizelement 6 ist als Dilatationsmedium vorzugsweise Luft vorgesehen. Erforderlichenfalls kann aber auch mit einer Flüssigkeit, z. B. Glyzerin, gearbeitet werden.

In Fig. 1 ist strichliniert bei dem Heizelement 6 ein Temperaturfühler 19 angedeutet, mittels dem die Heizelement-Temperatur gemessen werden kann. Der Temperatur-Fühler 19 ist an das Steuer- und Überwachungsgerät 9 über eine strichliniert angedeutete, galvanische Verbindung angeschlossen. Gegebenenfalls kann für den Temperaturfühler 19 einer der Stromversorgungs-Drähte 7 für das Heizelement 6 mitbenutzt werden. Das Steuer- und Überwachungsgerät 9 ist zweckmäßigerweise so ausgebildet, daß beispielsweise der Heizstrom 1, die Einwirkzeit t und, bei Verwendung eines Temperaturfühlers 19 auch die im Bereich des Heizelementes 6 herrschende Temperatur angezeigt und verarbeitet werden können. Bedarfsweise ist auch noch eine Anzeige für den Dilatationsdruck p vorgesehen.

Der Temperaturfühler kann als flächiges Teil innerhalb der Ballonwandung zusammen mit der Heizfolie 10 angeordnet sein.

Das äußere Katheterende 20 kann im Bereich der Spritze 4 mit einem insbesondere einstellbaren Sicherheitsventil 21 verbunden sein, daß bei Ausbildung des erfindungsgemäßen Katheters 1 als Dilatationskatheter und gleichzeitig als Heizkatheter auf die jeweils dabei vorgesehenen Drücke einstellbar ist.

Erwähnt sei noch, daß innerhalb des Dilatationsbereiches an dem Ballon 2 auch mehrere, gegebenenfalls voneinander unabhängige Heizelemente vorgesehen sein können, so daß innerhalb des vorgesehenen Wärmebehandlungsbereiches auch mit unterschiedlichen Temperaturen gearbeitet werden kann. Dies kann auch erreicht werden, indem die Heizfolie einen über ihre Längserstreckung unterschiedlichen, spezifischen elektrischen Widerstand aufweist, wobei die Leitfähigkeit zu den Enden des Ballons abnimmt und dort vorzugsweise in die Isolationsbereiche übergeht.

Auch kann durch Variation der Dicke der Heizfolie der Widerstand verändert und somit die Heizwirkung lokal bestimmt werden. Im Heizbereich kann die Heizfolie dabei vergleichsweise dünn mit höherem Widerstand ausgebildet sein und zu den Ballonenden hin in der Dicke und damit der Leitfähigkeit zunehmen. Die Heizfolie kann dann in etwas dickere oder breitere Folienbänder guter Leitfähigkeit übergehen, die dann gleichzeitig die galvanische Verbindung zur äußeren Stromquelle übernehmen können.

Die Figuren 7 bis 8 zeigen schematisierte Querschnittdarstellungen entsprechend den in Fig. 1 gezeigten Schnittlinien. Mit 22 ist dabei das Lumen oder die Innenhöhlung zum Zuführen des Dilatationsmediums zum Ballon-Lumen bezeichnet. In Figur 8 ist noch ein Durchtrittskanal 23 zur Druckmessung oder auch zum Zuführen von Kontrastmittel erkennbar.

Erwähnt sei noch, daß in den Figuren zur Verdeutlichung unmaßstäblich vergrößerte Darstellungen gewählt wurden. In Wirklichkeit beträgt der Außendurchmesser des Bal lons 2 in dilatiertem Zutand ein bis zu wenigen Millimetern und seine Länge ein bis zu mehreren Zentimetern. Bei einer Ballonlänge von z. B. 2 cm würde sich der beheizbare Mittelbereich vorzugsweise etwa über eine Länge von 1,5 cm erstrecken.

Für eine Kontrolle des Aufweitvorganges ist es vorteilhaft, wenn sich innerhalb des Ballons 2 ein Kontrastmittel befindet, das zweckmäßigerweise einem Dilatationsmedium zugegeben ist. Dadurch ist der Umriß des Ballons 2 durch ein bildgebendes Verfahren, z. B. Röntgen, gut erkennbar.

Alle in der Beschreibung, den Ansprüchen und der Zeichnung dargestellten Merkmale können sowohl einzeln als auch in beliebiger Kombination miteinander erfindungswesentlich sein.

## Ansprüche

1. Katheter mit einem am Endbereich befindlichen, dilatierbaren Ballon, der ein elektrisches Widerstands-Heizelement mit einer galvanischen Verbindung zu einer äußeren Stromquelle aufweist, wobei das Heizelement im Wandungsbereich des Ballons angeordnet ist, zum Erwärmen von dilatierten Gefäßabschnitten, **dadurch gekennzeichnet,** daß das Heizelement (6) eine elektrisch leitende Heizfolie aus Kunststoff ist, die nach außen, der Innenseite (13) eines Gefäßabschnittes (14) zugewandt, sowie nach innen elektrisch isoliert ist.

2. Katheter nach Anspruch 1, dadurch gekennzeichnet, daß die Heizzone des Ballons (2) im mittleren Bereich seiner Längserstreckung und mit Abstand zu seinen Enden vorgesehen ist und sich vorzugsweise über etwa zwei Drittel der Gesamtlänge erstreckt.

3. Katheter nach Anspruch 1 oder 2, dadurch gekennzeichnet, daß als galvanische Verbindung zwischen der Stromquelle (8) und dem Heizelement (6) Stromversorgungs-Drähte (7) vorgesehen sind, die vorzugsweise eine äußere Isolierung aufweisen und vorzugsweise innerhalb des Katheterschaftes geführt sind und daß insbesondere zwei oder gegebenenfalls auch eine davon vielfache Anzahl von Drähten (7) oder dgl. in der Katheterschaftwand integriert untergebracht sind.

4. Katheter nach Anspruch 1 oder 2, dadurch gekennzeichnet, daß als galvanische Verbindung Folienbänder (17) aus elektrisch leitendem Material, insbesondere aus Metallfolie oder leitender Kunststoffolie an oder vorzugsweise in der Katheterschaftwand vorgesehen sind.

5. Katheter nach einem oder mehreren der Ansprüche 1 bis 4, dadurch gekennzeichnet, daß gegenüberliegende Längskanten der Heizfolie (10) Anschlüsse (15) für die Enden der Stromversorgungs-Drähte, Folienbänder oder dgl. aufweisen, und daß die Anschlüsse bzw. Anschlußstellen insbesondere eine größere spezifische Leitfähigkeit als die Heizfolie selbst aufweisen.

6. Katheter nach einem oder mehreren der Ansprüche 1 bis 5, dadurch gekennzeichnet, daß die Heizfolie (10) oder dgl. als offener Ringmantel mit in Längsrichtung verlaufenden, voneinander getrennten Längskanten mit Anschlüssen für die Stromversorgungs-Drähte, Folienbänder oder dgl. ausgebildet sind, die vorzugsweise entlang den Anschlußkanten der Heizfolie 10) angeschlossen sind.

7. Katheter nach einem oder mehreren der Ansprüche 1 bis 5, dadurch gekennzeichnet, daß die Heizfolie (10) oder dgl. als geschlossener Ringmantel ausgebildet ist und daß die in Umfangsrichtung des Ballons (2) umlaufenden Ränder (16) der Heizfolie (10) als Anschlüsse für die Stromversorgungs-Drähte oder dgl. ausgebildet sind.

8. Katheter nach einem oder mehreren der Ansprüche 1 bis 7, dadurch gekennzeichnet, daß er als Heizballon-Katheter und gleichzeitig als Aufweitballon-Katheter ausgebildet und insbesondere bezüglich des Aufweitdruckes auf die Anforderungen beim Aufweiten bemessen ist.

9. Katheter nach einem oder mehreren der Ansprüche 1 bis 8, dadurch gekennzeichnet, daß innerhalb des Dilatationsbereiches an dem Ballon (2) mehrere, gegebenenfalls voneinander unabhängige Heizelemente vorgesehen sind.

10. Katheter nach einem oder mehreren der Ansprüche 1 bis 8, dadurch gekennzeichnet, daß die Heizfolie (10) oder dgl. einen über ihre Längserstreckung unterschiedlichen, spezifischen, elektrischen Widerstand aufweist.

11. Katheter nach einem oder mehreren der Ansprüche 1 bis 10, dadurch gekennzeichnet, daß die Heizfolie (10) einstückig in galvanische Verbindungen mit gegenüber der Heizfolie höherer Leitfähigkeit übergeht.

Fig.1

EP 0 315 982 A2

Fig. 2

Fig.5

Fig.6

Fig.4

Fig.3

Fig.8

Fig.7